# EUROPEAN PATENT APPLICATION

(11) **EP 4 438 605 A1**
(43) Date of publication of application: **02.10.2024**
(21) Application number: 22909131.9
(22) Date of filing: 17.05.2022
(51) Int. Cl.: C07D 487/04, A61K 31/53, A61P 31/14

(54) **CRYSTAL FORM OF NUCLEOSIDE COMPOUND**

(30) Priority: 23.12.2021 CN 202111593324
(71) Applicant: Shenzhen Antiv Pharma Co., Ltd., Shenzhen, Guangdong 518118 (CN)
(72) Inventor: LI, Shuo, Shenzhen, Guangdong 518118 (CN); LI, Guanguan, Shenzhen, Guangdong 518118 (CN); LIU, Xinjun, Shenzhen, Guangdong 518118 (CN); LI, Yingjun, Shenzhen, Guangdong 518118 (CN); ZHOU, Qifan, Shenzhen, Guangdong 518118 (CN)
(74) Representative: Herrmann, Uwe
(86) International application number: PCT/CN2022/093339
(87) International publication number: WO 2023/115795

(57) **Abstract**

The present invention provides a crystalline form of a nucleoside compound. The crystalline form is ATV014 crystalline form I, which has good solubility and thermal stability, and better bioavailability and dissolution profile in specific formulations. The good permeability and thermal stability of the crystalline form make it suitable for use in specific pharmaceutical formulations.

## Description

### TECHNICAL FIELD

The present invention belongs to the technical field of pharmaceuticals and relates to a crystalline form of a nucleoside compound.

### BACKGROUND

The novel coronavirus is an enveloped single-stranded RNA virus belonging to the β-genus coronavirus. Similar to SARS and MERS, the SARS-CoV-2 genome encodes non-structural proteins: 3-chymotrypsin-like protease (3CLpro), papain-like protease (PLpro), helicase, and RNA-dependent RNA polymerase (RdRp); structural proteins: such as spike glycoprotein and accessory proteins. The surface spike glycoprotein of the novel coronavirus binds to the angiotensin-converting enzyme (ACE2) receptor on the surface of human cells, thereby infecting the epithelial cells of the human respiratory tract. After the virus enters the host cell, it disassembles and releases the nucleocapsid and viral RNA into the cytoplasm. The 5' end open reading frame (ORF1a/b) of the viral RNA encodes polyproteins (ppla and pplab), which play a crucial role in the processing and maturation of enzymes required for viral replication, ppla and pplab can be cleaved by papain-like protease (PLpro) and 3-chymotrypsin-like protease (3CLpro) to produce non-structural proteins, including RNA-dependent RNA polymerase, helicase, etc., which are critical for the transcription and replication of the novel coronavirus. Currently, the surface spike glycoprotein of the novel coronavirus that recognizes the receptor, and the key proteins involved in the replication and transcription processes-3CLpro, PLpro, and RdRp-are four highly attractive targets for antiviral drug development.

Through the applicant's previous research on Remdesivir and its precursor compound GS-441524 (Li, et al., J. Med. Chem. 2020), it was found that GS-441524 exhibited superior antiviral activity compared to Remdesivir in *in vivo* activity tests in mice. Although the mechanism of action of the compound GS-441524 is similar to that of Remdesivir, it demonstrates better safety. Therefore, the applicant has filed a patent (application No. or patent No. 202011000517.2) describing the use of compound GS-441524 as a drug in the prevention, alleviation, and/or treatment of SARS-CoV-2.

The compound represented by the formula ATV014 is a derivative of the compound GS-441524. Through the inventor's research, it was found that the compound represented by the formula ATV014 exhibits better activity and bioavailability compared to the compound GS-441524.

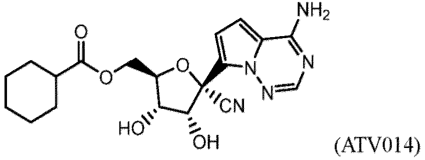

A drug may have different solid forms, and different solid forms of the same drug may exhibit significant differences in appearance, solubility, melting point, dissolution rate, bioavailability, and other aspects. These differences may also affect the stability, bioavailability, and efficacy of the drug. Therefore, it is necessary to have new solid forms of ATV014 with better physicochemical properties, particularly those with relatively high solubility, good stability, or high bioavailability.

### SUMMARY

### BRIEF SUMMARY

To solve the problems described above, the present invention provides the following technical solutions.

In a first aspect, a crystalline form of a compound represented by the formula (ATV014) is provided. The crystalline form has good physical stability and chemical stability, low hygroscopicity, which is beneficial for long-term storage, good solubility and thermal stability, and better bioavailability and dissolution profile in specific formulations. The good solubility and thermal stability of the crystalline form make it suitable for use in specific pharmaceutical formulations.

In a second aspect, a pharmaceutical composition comprising the aforementioned crystalline form is provided.

In a third aspect, use of the aforementioned crystalline form or the aforementioned pharmaceutical composition is provided.

In a fourth aspect, a method for preparing the aforementioned crystalline form is provided.

### DETAILED DESCRIPTION OF THE INVENTION

In a first aspect, a crystalline form of the compound represented by the formula (ATV014) is provided. In the present invention, the compound represented by the formula (ATV014) is named ATV014

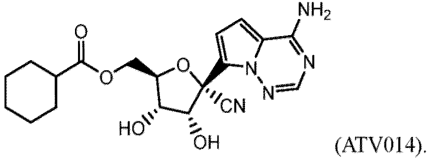

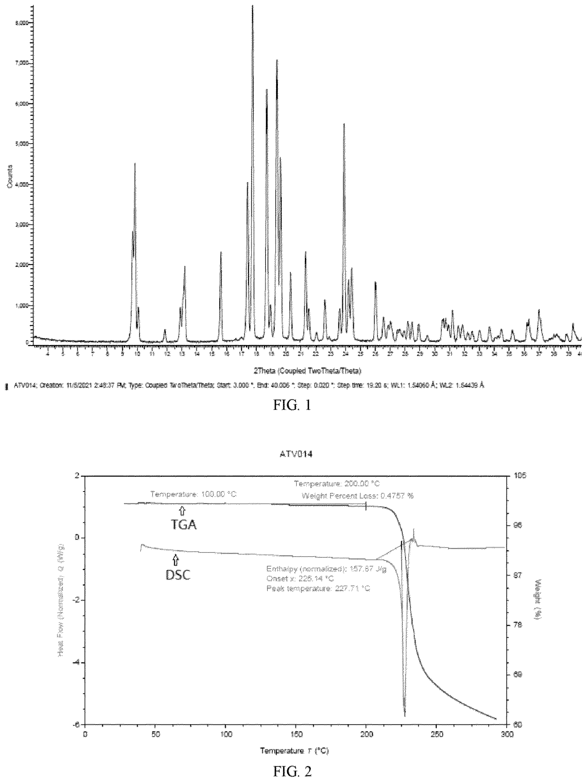

The present invention focuses on investigating whether ATV014 has various solid forms and has discovered the compound ATV014 crystalline form I. The X-ray powder diffraction pattern of the ATV014 crystalline form I is substantially as shown in FIG. 1. In some embodiments, the differential scanning calorimetry curve of the crystalline form I is substantially as shown by the differential scanning calorimetry curve in FIG. 2. In some embodiments, the thermogravimetric analysis curve pattern of the ATV014 crystalline form I is substantially as shown by the thermogravimetric analysis curve pattern in FIG. 2.

In some embodiments, the compound ATV014 crystalline form possesses at least one, or at least two, or all of the following characteristics (a)-(c): (a) The X-ray powder diffraction pattern of the crystalline form I is substantially as shown in FIG. 1; (b) the differential scanning calorimetry curve of the crystalline form I is substantially as shown by the differential scanning calorimetry curve in FIG. 2; and (c) the thermogravimetric analysis curve pattern of the crystalline form I is substantially as shown by the thermogravimetric analysis curve pattern in FIG. 2.

In some embodiments, the ATV014 crystalline form I possesses the following characteristics:
(a) The X-ray powder diffraction pattern of the crystalline form I is substantially as shown in FIG. 1;
(b) the differential scanning calorimetry curve of the crystalline form I is substantially as shown by the differential scanning calorimetry curve in FIG. 2; and
(c) the thermogravimetric analysis curve pattern of the crystalline form I is substantially as shown by the thermogravimetric analysis curve pattern in FIG. 2.

In some embodiments, the ATV014 crystalline form I has an XRPD spectrum that shows at least 2, at least 3, at least 4, at least 5, or at least 6 reflections at 2θ degrees, with the maximum intensity and the XRPD spectrum being substantially as shown in FIG. 1.

In some embodiments, the ATV014 crystalline form I exhibits characteristic peaks at XRPD diffraction angles 2θ of 9.69±0.2°, 9.84±0.2°, and 18.70±0.2°.

In some embodiments, the X-ray powder diffraction pattern of the ATV014 crystalline form I exhibits characteristic peaks at diffraction angles 2θ of 9.69±0.2°, 18.70±0.2°, and 23.88±0.2°, and exhibits characteristic peaks at one, two, or three of the diffraction angles 2θ of 9.84±0.2°, 17.74±0.2°, and 19.38±0.2°.

In some embodiments, the X-ray powder diffraction pattern of the ATV014 crystalline form I exhibits characteristic peaks at diffraction angles 2θ of 9.69±0.2°, 18.70±0.2°, and 23.88±0.2°, and exhibits characteristic peaks at one or two of the diffraction angles 2θ of 9.84±0.2°, 17.74±0.2°, and 19.38±0.2°.

In some embodiments, the X-ray powder diffraction pattern of the ATV014 crystalline form I exhibits characteristic peaks at diffraction angles 2θ of 9.69±0.2°, 9.84±0.2°, 17.74±0.2°, 18.70±0.2°, 19.38±0.2°, and 23.88±0.2°, and exhibits a characteristic peak at one of the diffraction angles 2θ of 13.19±0.2°, 15.61±0.2°, and 21.31±0.2°.

In some embodiments, the X-ray powder diffraction pattern of the ATV014 crystalline form I exhibits characteristic peaks at diffraction angles 2θ of 9.69±0.2°, 9.84±0.2°, 17.74±0.2°, 18.70±0.2°, 19.38±0.2°, and 23.88±0.2°, and exhibits characteristic peaks at two of the diffraction angles 2θ of 13.19±0.2°, 15.61±0.2°, and 21.31±0.2°.

In some embodiments, the X-ray powder diffraction pattern of the ATV014 crystalline form I exhibits characteristic peaks at diffraction angles 2θ of 9.69±0.2°, 9.84±0.2°, 13.91±0.2°, 15.61±0.2°, 17.74±0.2°, 18.70±0.2°, 19.38±0.2°, 21.31±0.2°, and 23.88±0.2°.

In some embodiments, the X-ray powder diffraction pattern of the ATV014 crystalline form I exhibits characteristic peaks at at least three of the diffraction angles 2θ of 9.69±0.2°, 9.84±0.2°, 13.91±0.2°, 15.61±0.2°, 17.74±0.2°, 18.70±0.2°, 19.38±0.2°, 21.31±0.2°, and 23.88±0.2°.

In some embodiments, the X-ray powder diffraction pattern of the ATV014 crystalline form I exhibits characteristic peaks at diffraction angles 2θ of 9.69±0.2°, 9.84±0.2°, 13.91±0.2°, 15.61±0.2°, 17.74±0.2°, 18.70±0.2°, 19.38±0.2°, 21.31±0.2°, and 23.88±0.2°, and exhibits characteristic peaks at one or both of the diffraction angles 2θ of 17.41±0.2° and 26.01±0.2°.

In some embodiments, the X-ray powder diffraction pattern of the ATV014 crystalline form I exhibits characteristic peaks at diffraction angles 2θ of 9.69±0.2°, 9.84±0.2°, 13.91±0.2°, 15.61±0.2°, 17.74±0.2°, 18.70±0.2°, 19.38±0.2°, 21.31±0.2°, and 23.88±0.2°, and exhibits a characteristic peak at one of the diffraction angles 2θ of 17.41±0.2° or 26.01±0.2°.

In some embodiments, the X-ray powder diffraction pattern of the ATV014 crystalline form I exhibits characteristic peaks at diffraction angles 2θ of 9.69±0.2°, 9.84±0.2°, 13.91±0.2°, 15.61±0.2°, 17.41±0.2°, 17.74±0.2°, 18.70±0.2°, 19.38±0.2°, 21.31±0.2°, 23.88±0.2°, and 26.01±0.2°.

In some embodiments, the X-ray powder diffraction pattern of the ATV014 crystalline form I exhibits characteristic peaks at at least three of the diffraction angles 2θ of 9.69±0.2°, 9.84±0.2°, 13.91±0.2°, 15.61±0.2°, 17.41±0.2°, 17.74±0.2°, 18.70±0.2°, 19.38±0.2°, 21.31±0.2°, 23.88±0.2°, and 26.01±0.2°.

In some embodiments, the differential scanning calorimetry curve of the ATV014 crystalline form I exhibits an endothermic peak at approximately 212 °C to approximately 238 °C. In some embodiments, the differential scanning calorimetry curve of the ATV014 crystalline form I exhibits an endothermic peak at approximately 220 °C to approximately 235 °C. In some embodiments, the differential scanning calorimetry curve of the ATV014 crystalline form I exhibits an endothermic peak with a peak value between 226-230 °C. In some embodiments, the differential scanning calorimetry curve of the ATV014 crystalline form I is substantially as shown in the differential scanning calorimetry curve graph in FIG. 2.

In some embodiments, the differential scanning calorimetry curve of the ATV014 crystalline form I shows a melting point of 225.14±5 °C.

In some embodiments, the thermogravimetric analysis curve of the ATV014 crystalline form I exhibits a weight loss of less than 1% at approximately 30 °C to approximately 200 °C. In some embodiments, the thermogravimetric analysis curve of the ATV014 crystalline form I exhibits a weight loss of less than 0.5% at 30-200 °C. In some embodiments, the thermogravimetric analysis curve of the ATV014 crystalline form I exhibits a weight loss of less than 1% at 100-200 °C. In some embodiments, the thermogravimetric analysis curve of the ATV014 crystalline form I exhibits a weight loss of less than 0.5% at 100-200 °C. In some embodiments, the thermogravimetric analysis curve of the ATV014 crystalline form I is substantially as shown in the thermogravimetric analysis curve graph in FIG. 2.

In some embodiments, the ATV014 crystalline form I is substantially pure.

The new crystalline form provided by the present invention has not been reported yet. The inventor of the present invention has conducted research and overcome this challenge, discovering a new crystalline form suitable for development. The new crystalline form has better stability and solubility, and a more stable crystalline form is of great significance for improving drug quality.

The new crystalline form provided by the present invention has good stability, is not prone to deliquescence under high humidity conditions, and is convenient for long-term storage of the drug. The crystalline form provided by the present invention has good stability and significant purification effects in the preparation process, which can effectively prevent crystal transformation during drug storage and development. This, in turn, avoids changes in bioavailability and efficacy, offering significant economic value.

In a second aspect, a pharmaceutical composition comprising the aforementioned crystalline form is provided.

Another objective of the present invention is to provide a pharmaceutical composition comprising a therapeutically effective amount of ATV014 crystalline form I and a pharmaceutically acceptable auxiliary material or excipient. Generally, the pharmaceutical composition or formulation is made by mixing or contacting a therapeutically effective amount of ATV014 crystalline form I with one or more pharmaceutical auxiliary materials, and the pharmaceutical composition or formulation is prepared in a manner well-known in the pharmaceutical field. The pharmaceutical composition or formulation can be used to treat diseases associated with viral infections, or in the prevention, alleviation, and/or treatment of symptoms associated with SARS-CoV-2-related diseases.

In some embodiments, a pharmaceutical composition comprises ATV014 free base, wherein at least 80% of the compound ATV014 free base is in the form of ATV014 crystalline form I. In some embodiments, a pharmaceutical composition comprises ATV014 free base, wherein at least 90% of the ATV014 free base is in the form of ATV014 crystalline form I. In some embodiments, a pharmaceutical composition comprises ATV014 free base, wherein at least 95% of the ATV014 free base is in the form of ATV014 crystalline form I. In some embodiments, a pharmaceutical composition comprises ATV014 free base, wherein at least 99% of the ATV014 free base is in the form of ATV014 crystalline form I.

In a third aspect, use of the aforementioned crystalline form or the aforementioned pharmaceutical composition is provided.

Provided is the use of the aforementioned crystalline form or the aforementioned pharmaceutical composition in the preparation of a drug for preventing, alleviating, and/or treating diseases or symptoms associated with viral infections.

The virus includes those selected from coronaviruses. In some embodiments, the virus includes those selected from SARS-CoV-2 or mutants thereof.

The present invention relates to the use of ATV014 crystalline form I, which can be used for treating diseases associated with viral infections, or in methods for preventing, alleviating, and/or treating symptoms associated with SARS-CoV-2-related diseases.

In a fourth aspect, a method for preparing the ATV014 crystalline form I is provided. The method comprises dissolving ATV014 in a good solvent, where the ATV014 may be in any solid form; adding an anti-solvent after complete dissolution to precipitate a crystal, followed by filtration and drying to obtain the crystalline form.

Dissolving ATV014 in the good solvent may be done by dissolving ATV014 in the good solvent at room temperature.

In some embodiments, the good solvent is selected from dimethyl sulfoxide, N-methylpyrrolidone, N,N-dimethylformamide, tetrahydrofuran, dichloromethane, methanol, or a combination thereof; and the anti-solvent is selected from water, methyl tert-butyl ether, or a combination thereof. In some embodiments, the good solvent is dimethyl sulfoxide, N-methylpyrrolidone, or a combination thereof, and the anti-solvent is water. In some embodiments, the good solvent is dimethyl sulfoxide, and the anti-solvent is water. In some embodiments, the good solvent is N-methylpyrrolidone, and the anti-solvent is water. In some embodiments, the good solvent is tetrahydrofuran, and the anti-solvent is water, methyl tert-butyl ether, or a combination thereof. In some embodiments, the good solvent is a mixed solvent of dichloromethane and methanol, and the anti-solvent is methyl tert-butyl ether.

### BENEFICIAL EFFECTS

Compared to the prior art, the present invention has the following technical effects:
(1) The ATV014 crystalline form I provided by the present invention does not undergo changes in its crystalline form or chemical purity when placed under hygroscopic conditions, demonstrating good physical and chemical stability.
(2) DVS results indicate that ATV014 crystalline form I has low hygroscopicity, which is advantageous for long-term storage.
(3) The ATV014 crystalline form I provided by the present invention has good solubility and thermal stability, and better bioavailability and dissolution profile in specific formulations. The good solubility and thermal stability of the crystalline form make it suitable for use in specific pharmaceutical formulations.

### DEFINITIONS

Unless otherwise specified, the following terms and phrases as used herein are intended to have the following meanings:
The term "crystalline form" refers to a unique ordered arrangement and/or conformation of molecules in the crystal lattice of a compound.

The term "substantially pure" refers to a crystalline form that is substantially free of one or more other crystalline forms. The crystalline form has a purity of at least 60%, or at least 70%, or at least 80%, or at least 85%, or at least 90%, or at least 93%, or at least 95%, or at least 98%, or at least 99%, or at least 99.5%, or at least 99.6%, or at least 99.7%, or at least 99.8%, or at least 99.9%, or it comprises other crystalline forms and the percentage of the other crystalline forms in the total volume or total weight of the crystalline form is less than 20%, or less than 10%, or less than 5%, or less than 3%, or less than 1%, or less than 0.5%, or less than 0.1%, or less than 0.01%.

The term "substantially free of" one or more other crystalline forms means that the content of other crystalline forms is less than 20%, or less than 10%, or less than 5%, or less than 4%, or less than 3%, or less than 2%, or less than 1%, or less than 0.5%, or less than 0.1%, or less than 0.01% of the total volume or weight.

The term X-ray powder diffraction pattern "substantially as shown in the figure" means that at least 50%, or at least 60%, or at least 70%, or at least 80%, or at least 90%, or at least 95%, or at least 99% of the peaks in the X-ray powder diffraction pattern appear in the figure.

The term "anti-solvent" refers to a solvent that can promote a solution to reach a state of supersaturation or crystallization. In some embodiments, the solubility of ATV014 in the anti-solvent is less than 0.001 g/L, or less than 0.01 g/L, or less than 0.1 g/L, or less than 0.2 g/L, or less than 0.3 g/L, or less than 0.4 g/L, or less than 0.5 g/L, or less than 0.6 g/L, or less than 0.8 g/L, or less than 1 g/L, or less than 2 g/L, or less than 3 g/L, or less than 4 g/L, or less than 5 g/L, or less than 6 g/L, or less than 7 g/L, or less than 8 g/L, or less than 9 g/L, or less than 10 g/L.

The term "peak or characteristic peak" in the context of patterns and/or data in figures refers to a feature that a person skilled in the art would not attribute to background noise.

The numbers in the present invention are all approximate values, regardless of whether terms like "about" or "approximately" are used. The numerical values may vary by 1%, 2%, 5%, 7%, 8%, 10%, 15%, or 20%, among others. Whenever a number with an N value is disclosed, any number with a value of N±1%, N±2%, N±3%, N±5%, N±7%, N±8%, N±10%, N±15%, or N±20% is explicitly disclosed, where "±" means plus or minus. Whenever a lower limit, R^{L}, and an upper limit, R^{U}, of a numerical range are disclosed, any numerical value within the disclosed range is explicitly disclosed. Specifically, the following numerical values explicitly disclosed within the range are included: R = R^{L} + K*(R^{U} - R^{L}), where K is a variable that increases in 1% increments from 1% to 100%, i.e., 1%, 2%, 3%, 4%, 5%, 50%, 51%, 52%, ... 95%, 96%, 97%, 98%, 99%, or 100%. In addition, the numerical ranges defined by the above two R numbers are also explicitly disclosed herein.

In the present invention, "room temperature" refers to ambient temperature, which ranges from approximately 10 °C to approximately 40 °C. In some embodiments, "room temperature" refers to a temperature range from approximately 20 °C to approximately 30 °C; in other embodiments, "room temperature" refers to a temperature range from approximately 25 °C to approximately 30 °C; and in yet other embodiments, "room temperature" refers to specific temperatures such as 10 °C, 15 °C, 20 °C, 25 °C, 30 °C, 35 °C, or 40 °C.

The term "treat" as used herein, unless otherwise indicated, means reversing, alleviating the condition or disease to which the term applies or one or more symptoms of such condition or disease, inhibiting the progression of the condition or disease or one or more symptoms thereof, or preventing the condition or disease or one or more symptoms thereof. The term "treatment" as used herein refers to the act of treating, as "treat" is defined above.

In the context of the present invention, "ATV014 crystalline form I", "ATV014 free base crystalline form I", "ATV014 crystalline form I" and "ATV014 free base crystalline form I", or ATV014 free base crystalline form I all denote the same meaning.

### BRIEF DESCRIPTION OF THE DRAWINGS

FIG. 1 shows the X-ray powder diffraction detection spectrum of ATV014 crystalline form I prepared according to an embodiment of the present invention;
FIG. 2 shows the differential scanning calorimetry (DSC) and thermogravimetric analysis (TGA) spectra of ATV014 crystalline form I according to an embodiment of the present invention;

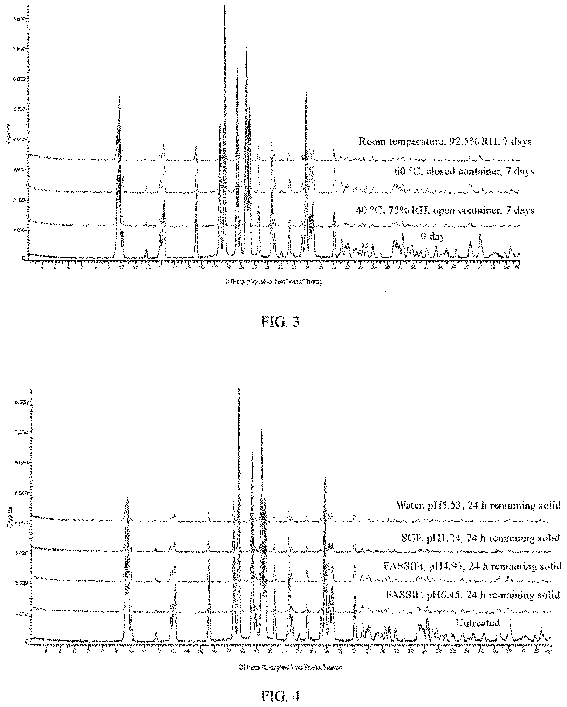

FIG. 3 shows the overlay XRPD patterns of stability test samples of ATV014 crystalline form I according to an embodiment of the present invention; and
FIG. 4 shows the overlay XRPD patterns of the remaining solid after solubility determination of ATV014 crystalline form I according to an embodiment of the present invention.

### DETAILED DESCRIPTION

In order to make the technical solutions of the present invention better understood by those skilled in the art, some non-limiting examples are further disclosed below to further explain the present invention in detail.

The reagents used in the present invention are either commercially available or can be prepared by the methods described herein.

### Instrument parameters

Unless otherwise specified in the parameters, all the following analyses are conducted at room temperature.

### X-ray Powder Diffraction Study

X-ray powder diffraction patterns were collected using a zero-background sample plate on a Bruker D8 Advance X-ray diffractometer equipped with an autosampler. The radiation source used was Cu Kα(λ=1.5418 Å), with the X-ray tube voltage set at 40 kV and the X-ray tube current set at 40 mA. The divergence slit for the X-rays was 0.6 mm. A suitable amount of the sample was placed in the center of the zero-background sample plate under the ambient condition and lightly pressed with a clean glass slide to obtain a smooth, flat surface. The zero-background sample holder was then fixed. Diffraction analysis of the sample was performed with a scan step size of 0.02° (2θ) over a range of 3-40° (2θ). The software used for data collection was DIFFRAC.COMMANDER, and the data were analyzed and presented using DIFFRAC.EVA.

### Differential Scanning Calorimetry (DSC)

DSC analysis of the sample was performed using a TA Insturment Discovery DSC 250 instrument with a lidded aluminum pan with a hole. Approximately 2-5 mg of the sample was weighed in the aluminum pan, sealed with a Tzero lid, and the sample amount was precisely recorded before transferring the sample to the instrument for measurement. The instrument was purged with nitrogen gas at 50 mL/min. Data were collected at a heating rate of 10 °C/min from 30 °C to 300 °C. The data were analyzed and presented using TRIOS.

### Thermogravimetric Analysis (TGA)

TGA analysis of the sample was performed using a TA Instrument Discovery TGA 55 instrument with an open aluminum pan. Typically, 2-5 mg of the sample was placed in a pre-balanced aluminum sample pan and heated from ambient temperature to 300 °C at 10 °C/min. The sample chamber was maintained with a nitrogen gas flow of 25 mL/min. In the TGA pattern, the x-axis represents temperature (°C), and the y-axis represents the percentage of weight loss (Weight (%)).

### Dynamic Vapor Sorption Analysis (DVS)

Vapor sorption/desorption data of the sample was obtained using DVS Intrinsic. A sample of 10-50 mg was weighed and placed in the sample chamber for automatic weighing. Parameters were set according to Table 1 to analyze the hygroscopicity of the sample.

**Table 1. DVS Parameter Settings**

| **Instrument** | SMS, DVS Intrinsic |
|---|---|
| **dm/dt** | 0.002%/min |
| **Sample amount** | 10 - 50 mg |
| **Drying/testing temperature** | 40 °C/ 25 °C |
| **Cycle** | Full cycle |
| **Minimum equilibrium time for dm/dt** | 30 min |
| **Maximum equilibrium time for dm/dt** | 120 min |
| **Data storage rate** | 5 s |
| **Gas and flow rate** | N₂, 200 sccm |
| **Post-run flow rate** | 200 sccm |
| **Step size** | 10% RH |
| **Method** | Sorption: 0, 10, 20, 30, 40, 50, 60, 70, 80, 90 |
| | Desorption: 80, 70, 60, 50, 40, 30, 20, 10, 0 |

### High-Performance Liquid Chromatography (HPLC) Detection of Crystalline Form

The instrument used for liquid chromatography analysis is the Agilent HPLC 1260 series.

**Table 2. HPLC Method for Stability Study**

| **Chromatographic column** | Agilent XDB-C18, 1.8 µm, 4.6*50 mm | | | |
|---|---|---|---|---|
| **Mobile phase** | A: 0.1% trifluoroacetic acid aqueous solution; B: 0.1% trifluoroacetic acid acetonitrile solution | | | |
| **Gradient (T/B%)** | Time | 0 min | 8 min | 10 min |
| | Mobile phase A | 90% | 25% | 0% |
| | Mobile phase B | 10% | 75% | 100% |
| **Column temperature** | 40 °C | | | |
| **Detector** | DAD detector, wavelength: 237 nm | | | |
| **Flow rate** | 1.0 mL/min | | | |
| **Injection volume** | 2.0 µL | | | |
| **Run time** | 10 min | | | |
| **Pre-run time** | 3 min | | | |
| **Diluent** | Acetonitrile: water = 1:1 | | | |

All the various dosage forms of the drug mentioned above can be prepared according to conventional methods in the pharmaceutical field.

In the present invention, the structures of some compounds represented by abbreviations are shown.

**Table 3**

| **Abbreviation** | **Chemical structure** |
|---|---|
| ATV-014 | 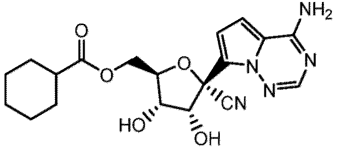 |

In describing the experimental details, certain abbreviations and acronyms were used. Although most of the abbreviations and acronyms can be understood by those skilled in the art, the following table contains a list of these abbreviations and acronyms. Table 4

| Abbreviation | Full version |
|---|---|
| ACN | Acetonitrile |
| DCC | Dicyclohexylcarbodiimide |
| DCM | Dichloromethane |
| DMAP | 4-Dimethylaminopyridine |
| EDMA | N,N-Dimethylethylamine |
| PE | Petroleum ether |
| rt | Room temperature |
| TEA | Triethylamine |
| TLC | Thin-layer chromatography |
| MeOH | Methanol |
| EtOH | Ethanol |
| IPA | Isopropanol |
| NBA | n-butanol |
| MEK | Butanone |
| MTBE | Methyl tert-butyl ether |
| EA | Ethyl acetate |
| IPAc | Isopropyl acetate |
| Tol | Toluene |
| Hept | n-Heptane |
| THF | Tetrahydrofuran |
| DMSO | Dimethyl sulfoxide |

The method for preparing the compound represented by the formula (ATV014) of the present invention can refer to the preparation methods disclosed in the prior art, such as the methods disclosed in Examples 1-3 of Chinese Patent CN2021106212456. Alternatively, the compound can be prepared according to the methods of the following examples.

### Example 1: Preparation of ATV014

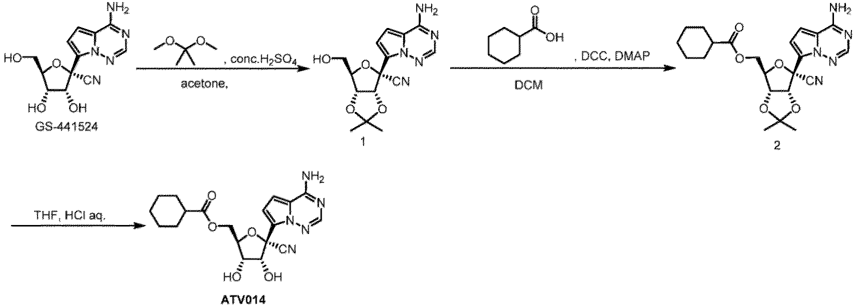

In a 500 mL reactor equipped with a stirrer, thermometer, and constant pressure dropping funnel, GS-441524 (10 g, 0.03 mol) and acetone dried with magnesium sulfate (300 mL) were added. Then 2,2-dimethoxypropane (17 g, 0.16 mol) was added. At room temperature, concentrated sulfuric acid (2.4 mL, 0.04 mol) was added dropwise to the system over 5 min. The solid began to dissolve. The mixture was heated to 45 °C and the reaction continued for 4 h. HPLC was used to monitor the completion of the reaction (OD-3 column, mobile phase: hexane/isopropanol = 80:20, flow rate: 0.8 mL/min, injection volume: 1 µL). Upon completion, the reaction was stopped, and the mixture was cooled in an ice bath. NaHCO₃ solid (10 g) and water (30 mL) were added to the reaction mixture. Sodium bicarbonate was used to adjust the pH to 7-8, and the mixture was distilled under reduced pressure to remove the solvent. The residue was diluted with ethyl acetate (300 mL). The ethyl acetate layer was washed with water (80 mL) and saturated brine (80 mL) separately, and dried over anhydrous sodium sulfate. The mixture was filtered under vacuum, and the filtrate was distilled under reduced pressure to about 100 mL of solvent remaining. The residue was slowly poured into petroleum ether cooled in an ice bath and stirred vigorously. A large amount of white solid was eluted out. 10.5 g of white solid compound 1 was obtained by filtration under vacuum with a yield of 91%.

150 g of Compound 1 was dissolved in 15 ml of dichloromethane, then cyclohexanecarboxylic acid and 554.0 mg of 4-dimethylaminopyridine were added. After stirring for 10 min, 10.2 g of dicyclohexylcarbodiimide was added, and the mixture was stirred at room temperature for 24 h. The mixture was separate by column chromatography (eluent: petroleum ether/ethyl acetate (V/V) = 1/1) to give Compound **2** (white solid). Compound **2** was dissolved in 30 mL of hydrochloric acid aqueous solution with a mass percentage of 37% and 150 mL of tetrahydrofuran. After stirring for 6 h, sodium carbonate was added to adjust the pH to 8. The organic solvent was removed by rotary evaporation to give ATV014 as a white solid.

### Example 2: Preparation of ATV014 Crystalline Form I

General Method: At room temperature, approximately 26 mg of ATV014 (sample prepared in Example 1) was added to a certain amount of solvent. After the sample was completely dissolved, a certain amount of anti-solvent (poor solvent) was slowly added dropwise to perform anti-solvent precipitation. The mixture was then filtered and dried to obtain the relevant ATV014 crystalline form I. The experimental results are shown in Table 1.

**Table 1: Anti-solvent Precipitation Experimental Results**

| **Batch No.** | **Solvent (mL)** | **Anti-solvent (mL)** | **XRPD result** |
|---|---|---|---|
| 41579-025-C1 | Dimethyl sulfoxide, 0.3 mL | Water, 0.6 mL | Crystalline form I |
| 41579-021-C2 | Dimethyl sulfoxide, 0.2 mL | Methyl tert-butyl ether, 0.2 mL | Layered, no solid |
| 41579-025-C3 | N-methylpyrrolidone, 0.5 mL | Water, 1 mL | Crystalline form I |
| 41579-025-C4 | N-methylpyrrolidone, 0.5 mL | Methyl tert-butyl ether, 1.4 mL | No solid |
| 41579-025-C5 | N,N-dimethylformamide, 0.3 mL | Water, 0.4 mL | Crystalline form I |
| 41579-025-C6 | N,N-dimethylformamide, 0.3 mL | Methyl tert-butyl ether, 1 mL | No solid |
| 41579-025-C7 | Tetrahydrofuran, 1 mL | Water, 1.4 mL | Crystalline form I |
| 41579-025-C8 | Tetrahydrofuran, 1 mL | Methyl tert-butyl ether, 1 mL | Crystalline form I |
| 41579-025-C9 | Dichloromethane:Methanol (1:1), 1.5 mL | Methyl tert-butyl ether, 1.4 mL | Crystalline form I |

ATV014 crystalline form I is an anhydrous crystalline form. The X-ray powder diffraction pattern of crystalline form I is shown in FIG. 1, and the complete absorption peaks are shown in Table 2. The differential scanning calorimetry curve of crystalline form I is substantially shown in FIG. 2. The thermogravimetric analysis curve pattern of crystalline form I is substantially shown in FIG. 2.

ATV014 crystalline form I, through the use of an X-ray powder diffractometer with Cu-Kα radiation, exhibits diffraction peaks at the following 2θ angles:

| 2θ angle (°) | Interplanar spacing (Å) | Peak intensity (Counts) | Relative peak intensity (%) |
|---|---|---|---|
| 9.691 | 9.11931 | 2928.67 | 37.90% |
| 9.838 | 8.98294 | 4953.17 | 65.00% |
| 10.079 | 8.76929 | 753.905 | 8.90% |
| 11.846 | 7.46461 | 329.858 | 3.30% |
| 12.891 | 6.86182 | 761.226 | 9.00% |
| 13.189 | 6.70774 | 1602.75 | 20.20% |
| 15.606 | 5.67367 | 1898.15 | 24.00% |
| 16.635 | 5.32498 | 160.317 | 0.70% |
| 16.982 | 5.21705 | 176.123 | 0.70% |
| 17.406 | 5.09071 | 3728.21 | 48.00% |
| 17.74 | 4.99573 | 7619.66 | 100.00% |
| 18.699 | 4.74164 | 6102.74 | 79.60% |
| 18.945 | 4.68044 | 822.83 | 9.00% |
| 19.38 | 4.5765 | 6371.75 | 83.20% |
| 19.615 | 4.52215 | 4226.28 | 54.60% |
| 20.302 | 4.37075 | 1351.17 | 16.40% |
| 21.311 | 4.16592 | 2230.36 | 28.40% |
| 21.525 | 4.12503 | 870.111 | 10.10% |
| 22.038 | 4.03013 | 259.506 | 1.90% |
| 22.615 | 3.92856 | 1123.54 | 13.40% |
| 22.887 | 3.88255 | 196.098 | 1.00% |
| 23.071 | 3.85203 | 167.13 | 0.60% |
| 23.596 | 3.76739 | 734.489 | 8.10% |
| 23.885 | 3.72259 | 5193.42 | 67.80% |
| 24.196 | 3.67534 | 1430.06 | 17.50% |
| 24.409 | 3.64375 | 1429.98 | 17.50% |
| 26.009 | 3.4231 | 1399.75 | 17.20% |
| 26.539 | 3.35599 | 598.003 | 6.40% |
| 26.859 | 3.31669 | 393.141 | 3.60% |
| 27.011 | 3.29835 | 544.035 | 5.60% |
| 27.487 | 3.24239 | 352.502 | 3.10% |
| 27.619 | 3.22714 | 417.641 | 4.00% |
| 27.933 | 3.19152 | 349.222 | 3.00% |
| 28.192 | 3.16283 | 530.33 | 5.50% |
| 28.46 | 3.13369 | 534.752 | 5.60% |
| 28.901 | 3.0868 | 580.422 | 6.30% |
| 29.486 | 3.02694 | 252.508 | 2.00% |
| 30.574 | 2.92162 | 435.405 | 4.40% |
| 30.73 | 2.90719 | 584.664 | 6.40% |
| 30.912 | 2.89044 | 462.345 | 4.70% |
| 31.175 | 2.86662 | 796.528 | 9.10% |
| 31.572 | 2.83151 | 440.237 | 4.40% |
| 31.85 | 2.80742 | 355.735 | 3.30% |
| 32.208 | 2.777 | 278.06 | 2.30% |
| 32.507 | 2.7522 | 310.504 | 2.80% |
| 33.001 | 2.71212 | 272.765 | 2.40% |
| 33.658 | 2.66063 | 480.48 | 5.10% |
| 34.067 | 2.62961 | 173.117 | 0.90% |
| 34.238 | 2.61686 | 221.595 | 1.60% |
| 34.455 | 2.60089 | 378.56 | 3.70% |
| 35.209 | 2.54694 | 311.832 | 2.90% |
| 35.484 | 2.52783 | 136.319 | 0.70% |
| 36.312 | 2.47206 | 417.582 | 4.30% |
| 36.983 | 2.4287 | 796.051 | 9.30% |
| 37.088 | 2.42205 | 288.178 | 2.50% |
| 37.691 | 2.38469 | 170.392 | 0.90% |
| 38.003 | 2.36582 | 234.051 | 1.80% |
| 38.175 | 2.35558 | 285.722 | 2.60% |
| 38.847 | 2.31634 | 237.046 | 2.00% |
| 39.284 | 2.29157 | 478.287 | 5.10% |
| 39.905 | 2.25735 | 227.045 | 1.10% |

### Example 3 Solid Stability Study

The ATV014 crystalline form I obtained from Example 2, batch number 41579-025-C1, was used for solid stability studies. The samples were placed under the conditions shown in Table 5, and then their XRPD and purity were tested. The experimental results are shown in **Table** , and the XRPD overlay patterns are shown in
**FIG..** After being placed for 7 days under conditions of 40 °C/75% RH, 60 °C, and 92.5% RH, the physical and chemical properties of ATV014 crystalline form I remained stable.

**Table 5. Determination Results for Stability of Crystalline Form I**

| **Crystalline form** | **Conditions** | **Time (days)** | **XRPD result** | **Purity %** |
|---|---|---|---|---|
| ATV014 free base crystalline form I | 0 day | 0 day | As shown in FIG. 3 | 98.12 |
| | 40 °C/75% RH, open container | 7 days | No change (as shown in FIG. 3) | 98.09 |
| | 60 °C, closed container | 7 days | No change (as shown in FIG. 3) | 98.10 |
| | Room temperature/92.5% RH, open container | 7 days | No change (as shown in FIG. 3) | 98.19 |

### Example 4. Solubility Determination

Based on the salt screening study results, the solubility of ATV014 crystalline form I obtained in Example 2, batch number 41579-025-C5, was determined at 37°C in FaSSIF, FeSSIF, SGF, and water at 0.5 h, 2 h, and 24 h. The solubility and pH values are shown in Table 6. The solubility of the sample is pH-dependent, meaning that the lower the pH, the higher the solubility. ATV014 crystalline form I has the highest solubility in SGF, approximately 2.9 mg/mL, and the lowest solubility in water, approximately 0.005 mg/mL. During the solubility determination process, the XRPD patterns of the remaining solids were consistent with that of ATV014 crystalline form I, indicating that the crystalline form did not change. The XRPD patterns are shown in **FIG..**

**Table 6. Solubility Test Results**

| **Sample** | **Medium** | **0.5 h** | | **2 h** | | **24 h** | | |
|---|---|---|---|---|---|---|---|---|
| | | **Solubility (mg/mL)** | **pH** | **Solubility (mg/mL)** | **pH** | **Solubility (mg/mL)** | **XPRD** | **pH** |
| Free base cry stalline form I | FaSSIF, pH 6.45 | 0.007 | 6.45 | 0.01 | 6.44 | 0.021 | Unchanged | 6.58 |
| | FeSSIF, pH 4.95 | 0.014 | 4.76 | 0.019 | 4.74 | 0.020 | Unchanged | 4.96 |
| | SGF, pH 1.24 | 2.48 | 0.95 | 2.56 | 0.9 | 2.86 | Unchanged | 1.26 |
| | Water, pH 5.53 | 0.005 | 7.24 | 0.006 | 6.45 | 0.005 | Unchanged | 6.5 |

### Example 5: Characterization Data Testing of ATV014 Crystalline Form I

The ATV014 crystalline form I obtained in Example 2 with batch number 41579-025-C5 was subjected to PLM (Polarized Light Microscopy Analysis), XRPD (X-ray Powder Diffraction), DSC (Differential Scanning Calorimetry), TGA (Thermogravimetric Analysis), and DVS (Dynamic Vapor Sorption Instrument) testing. The results are shown in Table 7.

**Table 7. Summary of Data for ATV014 Crystalline Form I**

| Crystalline form ^{a} | Solvation | PLM | XRPD | Crystallinity | DSC | TGA | DVS Hygroscopic weight gain % |
|---|---|---|---|---|---|---|---|
| ATV014 crystalline form I | Anhydrous crystalline form | Irregular; particle size 5-20 µm | As shown in FIG. 1 | High | As shown in the DSC curve in FIG. 2 | 0.4757% weight loss at 100-200 °C | 80% RH: 0.31%. |
| | | | | | | | 90% RH: 0.40%. |
| | | | | | | | Crystalline form unchanged after DVS testing |

| | | | | | | | |
|---|---|---|---|---|---|---|---|
| a) The purity of this batch of samples is 99.15%. | | | | | | | |

The method of the present invention has been described by preferred embodiments. It will be apparent to those skilled in the art that the method and application described herein can be implemented and applied with modification or with appropriate modification and combination within the content, spirit, and scope of the present invention. Those skilled in the art can modify the process parameters appropriately in view of the disclosure herein. It is specifically noted that all such substitutions and modifications will be apparent to those skilled in the art and are intended to be included herein.

## Claims

1. A crystalline form of ATV014, a structure of the crystalline form shown as formula ATV-014, 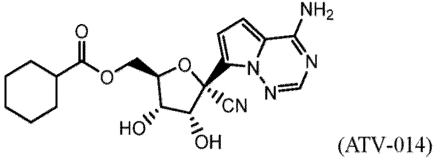 and the crystalline form of the ATV014 being crystalline form I, wherein an X-ray powder diffraction pattern of the ATV014 crystalline form I exhibits characteristic peaks at diffraction angles 2θ of 9.69±0.2°, 9.84±0.2°, 17.74±0.2°, 18.70±0.2°, 19.38±0.2°, and 23.88±0.2°.

2. The crystalline form of ATV014 according to claim 1, wherein the X-ray powder diffraction pattern of the ATV014 crystalline form I further exhibits characteristic peaks at one, two, or three of the diffraction angles 2θ of 13.19±0.2°, 15.61±0.2°, and 21.31±0.2°.

3. The crystalline form of ATV014 according to claim 1, wherein the X-ray powder diffraction pattern of the ATV014 crystalline form I further exhibits characteristic peaks at one or two of the diffraction angles 2θ of 13.19±0.2°, 15.61±0.2°, and 21.31±0.2°.

4. The crystalline form of ATV014 according to claim 1, wherein the X-ray powder diffraction pattern of the ATV014 crystalline form I exhibits characteristic peaks at the diffraction angles 2θ of 9.69±0.2°, 9.84±0.2°, 13.91±0.2°, 15.61±0.2°, 17.74±0.2°, 18.70±0.2°, 19.38±0.2°, 21.31±0.2°, and 23.88±0.2°.

5. The crystalline form of ATV014 according to claim 4, wherein the X-ray powder diffraction pattern of the ATV014 crystalline form I further exhibits characteristic peaks at one or both of the diffraction angles 2θ of 17.41±0.2° and 26.01±0.2°.

6. The crystalline form of ATV014 according to any one of claims 1-5, wherein the X-ray powder diffraction pattern of the ATV014 crystalline form I exhibits characteristic peaks at the diffraction angles 2θ of 9.69±0.2°, 9.84±0.2°, 13.91±0.2°, 15.61±0.2°, 17.41±0.2°, 17.74±0.2°, 18.70±0.2°, 19.38±0.2°, 21.31±0.2°, 23.88±0.2°, and 26.01±0.2°.

7. The crystalline form of ATV014 according to claim 1, wherein the X-ray powder diffraction pattern of the ATV014 crystalline form I is substantially as shown in FIG. 1.

8. The crystalline form of ATV014 according to claim 1, wherein a differential scanning calorimetry curve of the ATV014 crystalline form I exhibits an endothermic peak at 212-238 °C; or the differential scanning calorimetry curve of the ATV014 crystalline form I exhibits an endothermic peak at 220-235 °C; or the differential scanning calorimetry curve of the ATV014 crystalline form I exhibits an endothermic peak with a peak value at 226-230 °C; or the differential scanning calorimetry curve of the ATV014 crystalline form I is substantially as shown in the differential scanning calorimetry curve graph in FIG. 2.

9. The crystalline form of ATV014 according to claim 1, wherein a thermogravimetric analysis curve of the ATV014 crystalline form I exhibits a weight loss of less than 1% at 30-200 °C; or the thermogravimetric analysis curve of the ATV014 crystalline form I exhibits a weight loss of less than 0.5% at 30-200 °C; or the thermogravimetric analysis curve of the ATV014 crystalline form I is substantially as shown in the thermogravimetric analysis curve graph in FIG. 2.

10. A method for preparing the crystalline form of the ATV014 according to any one of claims 1-9, comprising dissolving the ATV014 in a good solvent, the ATV014 being in any solid form; adding an anti-solvent after complete dissolution to precipitate a crystal, followed by filtration and drying to obtain the ATV014 crystalline form I.

11. The preparation method according to claim 10, wherein the good solvent comprises a solvent selected from dimethyl sulfoxide, N-methylpyrrolidone, N,N-dimethylformamide, tetrahydrofuran, dichloromethane, methanol, or a combination thereof; and the anti-solvent comprises a solvent selected from water, methyl tert-butyl ether, or a combination thereof.

12. The preparation method according to claim 10, wherein the good solvent is dimethyl sulfoxide, N-methylpyrrolidone, or a combination thereof, and the anti-solvent is water; or the good solvent is tetrahydrofuran, and the anti-solvent is water or methyl tert-butyl ether or a combination thereof; or the good solvent is a mixed solvent of dichloromethane and methanol, and the anti-solvent is methyl tert-butyl ether.

13. A pharmaceutical composition, comprising a therapeutically effective amount of the compound ATV014 crystalline form according to any one of claims 1-9, and a pharmaceutically acceptable excipient.

14. Use of the crystalline form of the compound ATV014 according to any one of claims 1-9 or the pharmaceutical composition according to claim 13 in the preparation of a drug for preventing, alleviating, and/or treating diseases or symptoms associated with viral infections.
